# EUROPEAN PATENT SPECIFICATION

(11) **EP 4 448 082 B1**
(45) Date of publication and mention of the grant of the patent: **04.11.2026**
(21) Application number: 21841143.7
(22) Date of filing: 16.12.2021
(51) Int. Cl.: A61N 1/36, A61N 1/375, A61N 1/05, A61N 1/372

(54) **COCHLEAR IMPLANTS HAVING MRI-COMPATIBLE MAGNET APPARATUS**
COCHLEAIMPLANTATE MIT MRT-KOMPATIBLER MAGNETVORRICHTUNG
IMPLANTS COCHLÉAIRES AYANT UN APPAREIL À AIMANT COMPATIBLE IRM

(43) Date of publication of application: 23.10.2024
(73) Proprietor: Advanced Bionics LLC, Valencia, CA 91355 (US)
(72) Inventor: SMITH, James George Elcoate, Santa Clarita, CA 91350 (US); LEE, Sung Jin, Valencia, CA 91354 (US)
(74) Representative: Schwan Schorer & Partner mbB
(86) International application number: PCT/US2021/063657
(87) International publication number: WO 2023/113790

(56) References cited:
- WO-A1-2013/043176
- WO-A1-2014/164023
- WO-A1-2017/087004
- WO-A1-2017/105604
- US-A1- 2018 146 308

## Description

### BACKGROUND

### 1. Field

The present disclosure relates generally to implantable cochlear stimulation (or "ICS") systems.

### 2. Description of the Related Art

ICS systems are used to help the profoundly deaf perceive a sensation of sound by directly exciting the intact auditory nerve with controlled impulses of electrical current. Ambient sound pressure waves are picked up by an externally worn microphone and converted to electrical signals. The electrical signals, in turn, are processed by a sound processor, converted to a pulse sequence having varying pulse widths, rates and/or amplitudes, and transmitted to an implanted receiver circuit of the ICS system. The implanted receiver circuit is connected to an implantable electrode array that has been inserted into the cochlea of the inner ear, and electrical stimulation current is applied to varying electrode combinations to create a perception of sound. The electrode array may, alternatively, be directly inserted into the cochlear nerve without residing in the cochlea. A representative ICS system is disclosed in U.S. Patent No. 5,824,022, which is entitled "Cochlear Stimulation System Employing Behind-The-Ear Sound processor With Remote Control". Examples of commercially available ICS sound processors include, but are not limited to, the Harmony ^{™} BTE sound processor, the Naida^{™} CI Q Series sound processor and the Neptune^{™} body worn sound processor, which are available from Advanced Bionics.

As alluded to above, some ICS systems include an implantable cochlear stimulator (or "cochlear implant"), a sound processor unit (e.g., a body worn processor or behind-the-ear processor), and a microphone that is part of, or is in communication with, the sound processor unit. The cochlear implant communicates with the sound processor unit and, some ICS systems include a headpiece that is in communication with both the sound processor unit and the cochlear implant. The headpiece communicates with the cochlear implant by way of a transmitter (e.g., an antenna) on the headpiece and a receiver (e.g., an antenna) on the implant. Optimum communication is achieved when the transmitter and the receiver are aligned with one another. To that end, the headpiece and the cochlear implant may include respective positioning magnets that are attracted to one another, and that maintain the position of the headpiece transmitter over the implant receiver. The implant magnet may, for example, be located within a pocket in the cochlear implant housing. The skin and subcutaneous tissue that separates the headpiece magnet and implant magnet is sometimes referred to as the "skin flap," which is frequently 3 mm to 11 mm thick.

The present inventors have determined that conventional cochlear implants are susceptible to improvement. For example, cochlear implants with magnet apparatus that are compatible with magnetic resonance imaging ("MRI") systems have be introduced in recent years. The MRI-compatible magnet apparatus have a case defining a central axis, a frame within the case that is rotatable relative to the case about the central axis, and a plurality of elongate diametrically magnetized magnets that are located in the frame and that are rotatable about their respective longitudinal axis relative to the frame. This combination allows the magnets to align with three-dimensional (3D) MRI magnetic fields, regardless of field direction.

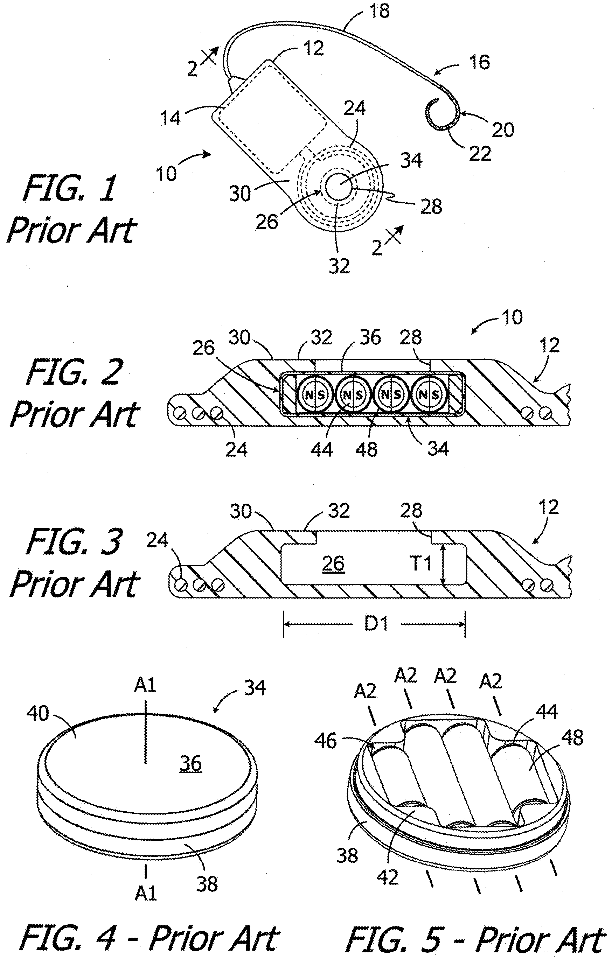

One example of a conventional cochlear implant (or "implantable cochlear stimulator") is illustrated in FIGS. 1-5. Cochlear implant 10 includes a flexible housing 12 formed from a silicone elastomer or other suitable material, a processor assembly 14, a cochlear lead 16 with a flexible body 18 and an electrode array 20 with electrodes 22 (e.g., platinum electrodes), and an antenna 24 that may be used to receive data and power by way of an external antenna that is associated with, for example, a sound processor unit. The housing 12 also includes a pocket 26, an opening 28, and a top wall 30. An MRI-compatible magnet apparatus (as shown), or a positioning magnet, is located in the pocket 26 and used to maintain the position of a headpiece transmitter over the antenna 22. The opening 28 extends through the top wall 30 to the magnet pocket 26. The portion of the top wall 30 between the opening 28 and the outer edge of the magnet apparatus or positioning magnet forms a retainer 32 that, absent deformation of the aperture and retainer, prevents the magnet or magnet apparatus from coming out of the housing 12.

The cochlear implant 10 also includes a MRI-compatible magnet apparatus (or "magnet assembly") 34 within the pocket 26. The magnet apparatus 34 includes a case 36, with a base 38 and a cover 40, a frame 42 that is rotatable relative to the case about axis A1, and four elongate diametrically magnetized magnets 44 located within the frame receptacle 46 that are rotatable relative to the frame about their respective longitudinal axes A2. As compared to one another, two of the magnets 44 are relatively long and two of the magnets are relatively short. The magnets 44 are located within tubes 48 formed from low friction material. Additional examples of such MRI-compatible magnet apparatus may be found in U.S. Pat. Nos. 9,919,154, 10,463,849, and 10,532,209. Another proposed magnet apparatus, which includes a single elongate magnet, is described in PCT Pat. Pub. No. 2020/092185 A1.

Although such MRI-compatible magnet apparatus have proven to be a significant advance in the art, the present inventors have determined that they are susceptible to improvement. For example, the use of a rotating frame and a plurality of elongate diametrically magnetized magnets within the frame results in less magnetic material within the case as compared to a solid, disk-shaped magnet. The benefits of MRI-compatibility far outweigh any magnetic strength issues associated with the reduction in magnetic material for most recipients. For some recipients, however, post-implantation increases in the distance between the implanted magnet apparatus and the headpiece magnet can be problematic because of the corresponding decrease in magnetic attraction force between the cochlear implant and the headpiece. The increase in distance may be the result of an increase in skin flap thickness due to significant weight gain or an increase in hair thickness in the area of the cochlear implant.

The present inventors have also determined that some recipients who require a relatively weak MRI-compatible magnet apparatus at the time of implantation may require a stronger MRI-compatible magnet apparatus at a later date. For example, a relatively weak MRI-compatible magnet apparatus that is appropriate for an infant with a thin and fragile skin flap may not be ideal for an adult who requires a stronger MRI-compatible magnet apparatus due to an increase in skin flap thickness.

WO 2013/043176 A1 relates to a cochlear implant including a non-magnetic nest embedded within an encapsulant in a non-removable manner; also an antenna is embedded within the encapsulant. The embedded nest is for selectively engage or disengage a magnet case of an attachment magnet, so that the magnet case with the magnet can be removed by surgery before performing an MRI procedure. WO 2017/105605 A1 relates to cochlear implant including a case for housing a rotatable non-magnetic frame with receptacles for receiving a plurality of rotatable diametrically magnetized cylindrical magnets.

### SUMMARY

The invention relates to a cochlear implant as defined in claim 1.

There are a number of advantages associated with such apparatus. By way of example, but not limitation, the present apparatus facilitate in-situ post-implantation retrofit procedures that may be used to increase the magnet strength in those instances where there increases in skin flap thickness necessitate or permit an increase in magnetic strength of the cochlear implant.

The above described and many other features of the present inventions will become apparent as the inventions become better understood by reference to the following detailed description when considered in conjunction with the accompanying drawings.

### BRIEF DESCRIPTION OF THE DRAWINGS

Detailed descriptions of the exemplary embodiments will be made with reference to the accompanying drawings.
FIG. 1 is a top view of a cochlear implant.
FIG. 2 is a partial section view taken along line 2-2 in FIG. 1.
FIG. 3 is a section view of a portion of the cochlear implant illustrated in FIG. 1.
FIG. 4 is a perspective view of a portion of the cochlear implant illustrated in FIG. 1.
FIG. 5 is a perspective view of a portion of the cochlear implant illustrated in FIG. 1.
FIG. 6 is a side view of an implant magnet system in accordance with one embodiment of a present invention.
FIG. 7 is a section view taken along line 7-7 in FIG. 6.
FIG. 8 is a perspective view of the implant magnet system illustrated in FIG. 6.
FIG. 9 is an exploded perspective view of the implant magnet system illustrated in FIG. 6.
FIG. 10 is a section view of a portion of the cochlear implant illustrated in FIGS. 1 and 2 with the implant magnet system illustrated in FIGS 6-9 in place of the magnet apparatus illustrated in FIGS. 1 and 2.
FIG. 10A is a flow chart showing a method in accordance with one embodiment of a present invention.
FIG. 11 is a section view of a portion of a cochlear implant.
FIG. 12 is a section view of the portion of the cochlear implant illustrated in FIG. 11 with the magnet apparatus removed.
FIG. 13 is a side view of an implant magnet system in accordance with one embodiment of a present invention.
FIG. 14 is a top view of the implant magnet system illustrated in FIG. 13.
FIG. 15 is an exploded perspective view of the implant magnet system illustrated in FIG. 13.
FIG. 16 is an exploded perspective view of a portion of the implant magnet system illustrated in FIG. 13.
FIG. 17 is partial section view of the implant magnet system illustrated in FIG. 13.
FIG. 18 is a section view of a portion of the cochlear implant illustrated in FIG. 11 with the implant magnet system illustrated in FIGS 13-17 in place of the magnet apparatus illustrated in FIG. 11.
FIG. 18A is a flow chart showing a method in accordance with one embodiment of a present invention.
FIG. 19 is an exploded perspective view of an implant magnet system in accordance with one embodiment of a present invention.
FIG. 20 is a side view of a portion of the implant magnet system illustrated in FIG. 19.
FIG. 21 is a perspective view of a portion of the implant magnet system illustrated in FIG. 19.
FIG. 22 is a section view of the implant magnet system illustrated in FIG. 19.
FIG. 22A is a section view of a portion of the cochlear implant illustrated in FIG. 11 with the implant magnet system illustrated in FIGS 19-22 in place of the magnet apparatus illustrated in FIG. 11.
FIG. 22B is a flow chart showing a method in accordance with one embodiment of a present invention.
FIG. 23 is a perspective view of an implant magnet apparatus in accordance with one embodiment of a present invention.
FIG. 24 is a perspective view of a portion of the implant magnet apparatus illustrated in FIG. 23.
FIG. 25 is a perspective view of an implant magnet apparatus in accordance with one embodiment of a present invention.
FIG. 26 is a perspective view of a portion of the implant magnet apparatus illustrated in FIG. 25.
FIG. 27 is a perspective view of an implant magnet apparatus in accordance with one embodiment of a present invention.
FIG. 28 is a perspective view of a portion of the implant magnet apparatus illustrated in FIG. 27.
FIG. 29 is a perspective view of an implant magnet apparatus in accordance with one embodiment of a present invention.
FIG. 30 is a perspective view of a portion of the implant magnet apparatus illustrated in FIG. 29.
FIG. 31 is a block diagram of a cochlear implant system in accordance with one embodiment of a present invention.

### DETAILED DESCRIPTION OF THE EXEMPLARY EMBODIMENTS

The following is a detailed description of the best presently known modes of carrying out the inventions. This description is not to be taken in a limiting sense, but is made merely for the purpose of illustrating the general principles of the inventions.

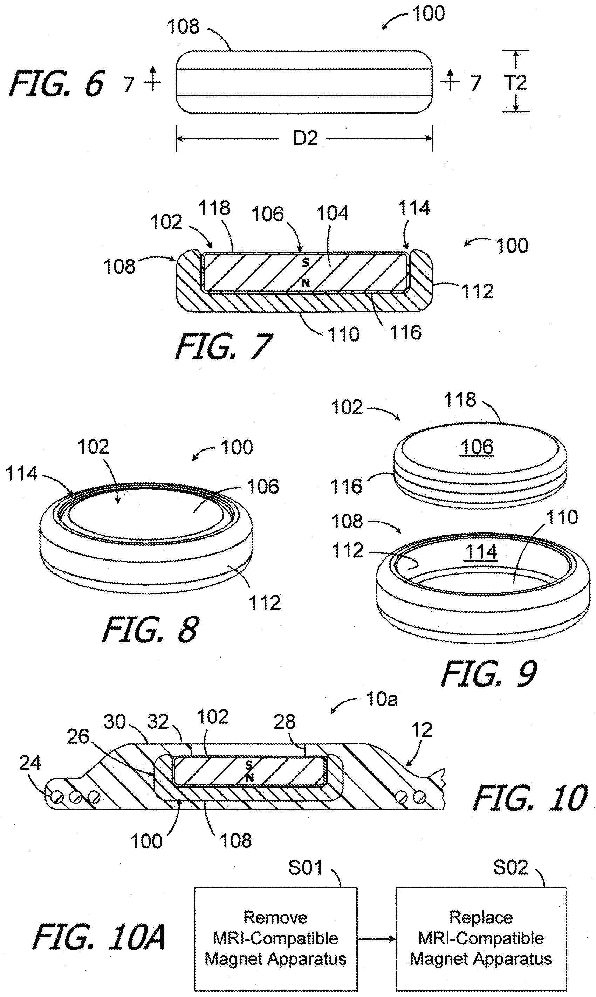

One example of a cochlear implant that may benefit from a post-implantation in-situ retrofit (i.e., a retrofit that is performed while the cochlear implant is and remains implanted in the recipient) in accordance with the present inventions is the cochlear implant 10 described above with references to FIGS. 1-5, and one example of a magnet system that may be used to increase the magnetic strength of a cochlear implant such as, for example, the cochlear implant 10, is the magnet system 100 illustrated in FIGS. 6-9. The exemplary magnet system 100 includes a positioning magnet 102, with a magnetic disk 104 and a case 106, and a non-magnetic spacer (or "spacer") 108. The positioning magnet 102 and spacer 108 together define an overall disk-like shape. To that end, the spacer 108 includes a base 110 and an annular side wall 112 that together define a receptacle 114. The inner diameter of the side wall 112 is substantially equal to the outer diameter of the positioning magnet 102 (as defined by the case 106), while the depth of the receptacle 114 is substantially equal to the thickness of the positioning magnet 102 (as defined by the case 106). The positioning magnet 102 may be removable from the spacer 108, or may be permanently secured thereto through the use of, for example, laser welding or a biocompatible adhesive that secures the case 106 to the spacer. As used herein, the phrase "permanently secured" means that, once connected, the positioning magnet and spacer will remain secured to one another under normal use conditions, and cannot be separated from one another without destruction of the positioning magnet, the spacer and/or the instrumentality that secures the two to one another. It should also be noted here that the cases and spacers described herein are not part of the cochlear implant housing in which they are located.

The size and shape of the magnet system 100 may be substantially equal to the cochlear implant magnet pocket that it is intended to be inserted into during, for example, an in-situ retrofit. Turning to FIG. 10, the exemplary magnet system 100 in the exemplary cochlear implant 10a may be the same size, or essentially the same size, as the pocket 26. As such, the magnet system 100 may be used in an in-situ retrofit procedure where, for example, it is desired to replace a magnet apparatus such as magnet apparatus 34 with four rotating magnets 44 (FIGS. 1-5) with the positioning magnet 102 despite the fact that the positioning magnet 102 is smaller than both the pocket 26 and the removed magnet apparatus 34. Referring to FIG. 10A, the magnet apparatus 34 may be removed from the pocket 26 in-situ by way of the opening 28 in the housing 12 (Step 01). The removal may be accomplished by temporarily deforming the opening 28 and retainer 32 and without removing portions of the housing or otherwise damaging the housing 12. The magnet system 100 may then be installed into the same pocket 26 in place of the removed magnet apparatus 34 by way of the same opening 28 (Step 02). The replacement may also be accomplished by temporarily deforming the opening 28 and retainer 32 and without removing portions of the housing or otherwise damaging the housing 12. Suitable removal and installation tools and techniques are illustrated and described in U.S. Pat. No. 10,124,167.

In some implementations, the diameter D1 of the pocket 26 (FIGS. 2 and 3) may range from 9 mm to 16 mm and the thickness T1 may range from 1.5 mm to 3.0 mm and, in the illustrated embodiment, the diameter D1 of the pocket 26 is 12.9 mm and the thickness T1 is 2.4 mm. Accordingly, in the illustrated embodiment, the diameter D2 of the magnet system 100 (FIG. 6) may range from about 9 mm to about 16 mm and the thickness T2 may range from about 1.5 mm to about 3.0 mm and, in the illustrated embodiment, the diameter D2 of the magnet system 100 is about 12.9 mm and the thickness T2 is about 2.4 mm. As used herein in the context of the magnet system 100, the word "about" means ± 10%.

The magnet 104 in the exemplary magnet system 100 is an axially magnetized disk-shaped magnet. Diametrically magnetized disk-shaped magnets may also be employed. Suitable materials for the magnet 104 include, but are not limited to, neodymium-boron-iron and samarium-cobalt.

The exemplary case 106 is not limited to any particular configuration, size or shape. In the illustrated implementation, the case 106 is a two-part structure that includes a base 116 and a cover 118 (FIGS. 7 and 9) which are secured to one another in such a manner that a hermetic seal is formed between the cover and the base. Suitable techniques for securing the cover 118 to the base 116 include, for example, seam welding with a laser welder. With respect to materials, the case 106 may be formed from biocompatible paramagnetic metals, such as titanium or titanium alloys, and/or biocompatible non-magnetic plastics such as polyether ether ketone (PEEK), low-density polyethylene (LDPE), high-density polyethylene (HDPE), ultra-high-molecular-weight polyethylene (UHMWPE), polytetrafluoroethylene (PTFE) and polyamide. In particular, exemplary metals include commercially pure titanium (e.g., Grade 2) and the titanium alloy Ti-6Al-4V (Grade 5), while exemplary metal thicknesses may range from 0.20 mm to 0.25 mm. With respect to size and shape, the case 106 may have an overall size and shape similar to the cases of conventional cochlear implant positioning magnets.

Suitable materials for the exemplary spacer 108, which may be formed by machining, metal injection molding or injection molding, include paramagnetic metals, polymers and plastics such as those discussed above in the context of the case 106. A coating such as a diamond-like carbon (DLC) coating that reduces the likelihood of biofilm formation may be applied to the spacer 108.

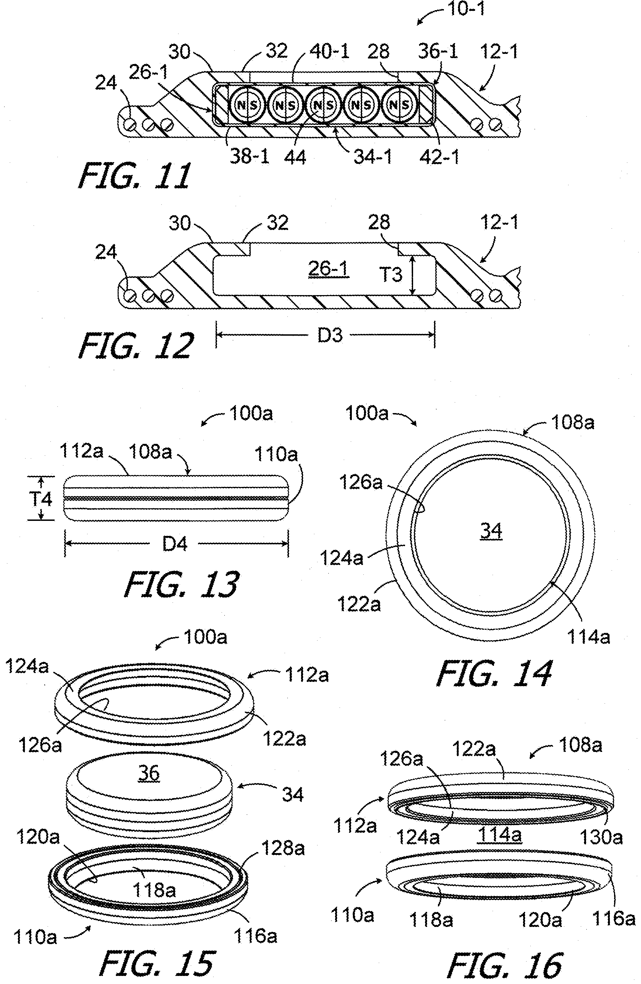

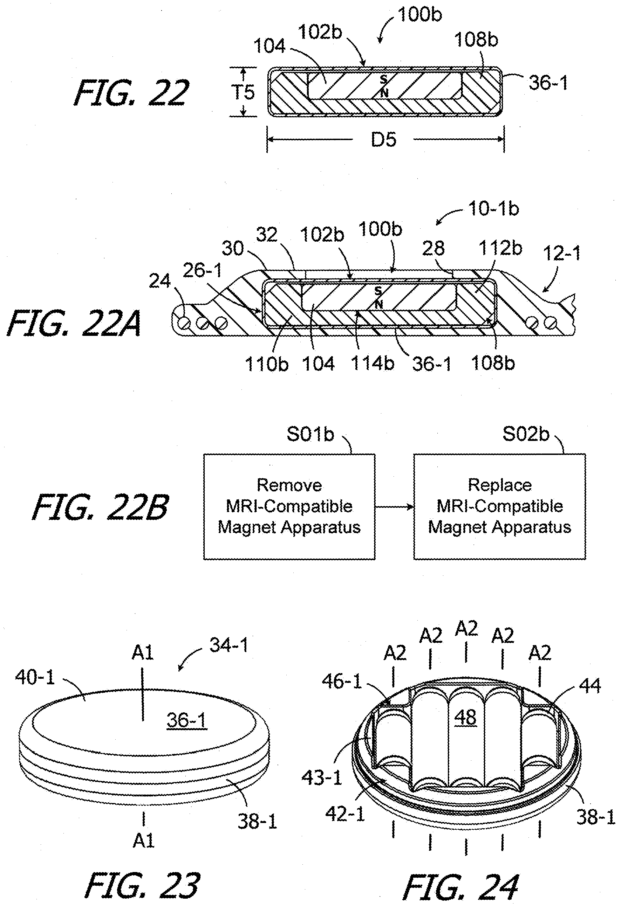

In some situations, magnet systems may be used to reduce the magnetic strength of an implanted cochlear implant. Referring first to FIGS. 11 and 12, the exemplary cochlear implant 10-1 is substantially similar to cochlear implant 10 and similar elements are represented by similar reference numerals. For example, the cochlear implant 10-1 includes a flexible housing 12-1, a processor assembly and a cochlear lead (such as those discussed above with reference to FIG. 1), and an antenna 24. Here, however, the cochlear implant 10-1 includes an MRI-compatible magnet apparatus 34-1 that is larger than the magnet apparatus 34 as well as a pocket 26-1 that is larger than the pocket 26 to accommodate the larger magnet apparatus. The magnet apparatus 34-1, which is discussed in greater detail below with reference to FIGS. 23 and 24, includes a case 36-1, with a base 38-1 and a cover 40-1, a frame 42-1 that is rotatable relative to the case, and five elongate diametrically magnetized magnets 44 located within the frame. The magnet apparatus 34-1 is larger than the magnet apparatus 34 and, accordingly, the magnet pocket 26-1 is larger than the pocket 26. In some instances, the diameter D3 of the pocket 26-1 may range from 9.4 mm to 17.4 mm and the thickness T3 may range from 1.5 mm to 4.0 mm and, in the illustrated embodiment, the diameter D3 is 12.6 mm and the thickness T3 is 3.1 mm.

The exemplary magnet system 100a illustrated in FIGS. 13-17, which may take the place of the magnet apparatus 34-1 in the cochlear implant 10-1 before or after implantation depending upon circumstances, includes the above-described magnet apparatus 34 with only four elongate diametrically magnetized magnets as well as a non-magnetic spacer (or "spacer") 108a. The exemplary spacer 108a includes a bottom portion 110a and a top portion 112a that together define a receptacle 114a for the magnet apparatus 34. The bottom portion 110a includes a side wall 116a and a flange 118a that defines an opening 120a, while the top portion 112a includes a side wall 122a and a flange 124a that defines an opening 126a. The inner diameters of the openings 120a and 126a are smaller than the outer diameters of the adjacent portions of the magnet apparatus 34, which prevents the magnet apparatus from passing through the openings. The exemplary bottom and top portions 110a and 112a may be aligned with one another by way of, for example a recess 128a on the bottom portion 110a and a projection 130a on the top portion 112a. The magnet apparatus 34-1 may be removable from the spacer 108a, or may be permanently secured thereto by securing the bottom and top portions 110a and 112a to one another with, for example, laser welding or a biocompatible adhesive.

Suitable materials for the exemplary spacer 108a, which may be formed by machining, metal injection molding or injection molding, include paramagnetic metals, polymers and plastics such as those discussed above in the context of the case 106. A coating that reduces the likelihood of biofilm formation (e.g., a DLC coating) may be applied to the spacer 108a.

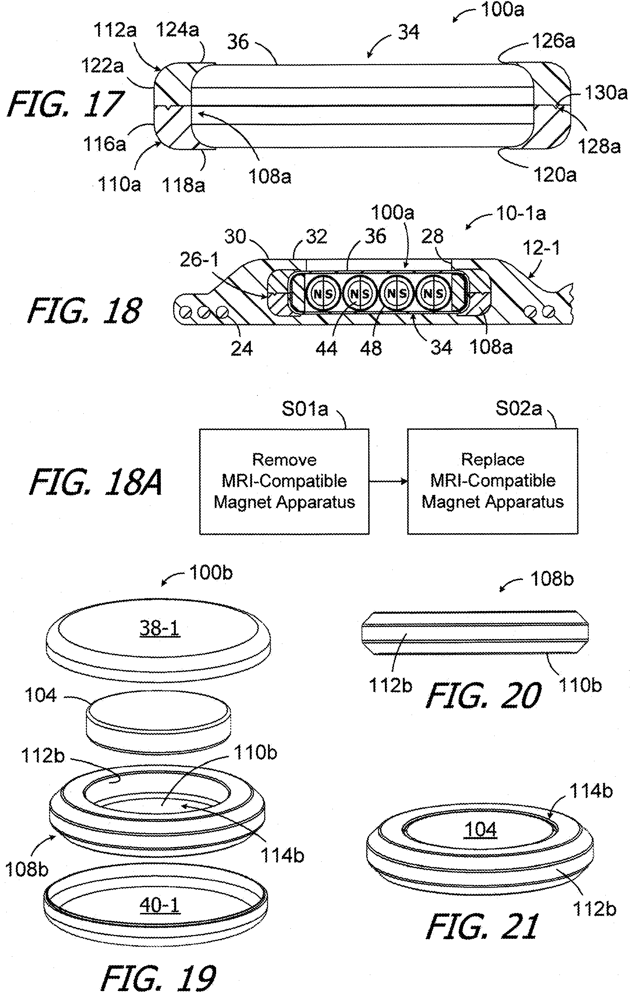

Turning to FIGS. 18 and 18A, and as alluded to above, the magnet system 100a may be inserted into the cochlear implant housing 12-1 to form the cochlear implant 10-1a. This may be done prior to implantation in, for example, instances where the it may be desirable to provide the recipient with a cochlear implant that has a relatively low magnetic strength MRI-compatible magnet apparatus at the time of implantation (e.g., when the cochlear implant is implanted into an infant) and that can be retrofit in-situ to a higher magnetic strength MRI-compatible magnet apparatus at a later time if necessary (e.g., when the recipient is an adult). For example, the magnet system 100a with magnet apparatus 34 (FIG. 18) may be removed from the pocket 26-1 in-situ by way of the opening 28 in the housing 12-1 (Step 01a). The removal may be accomplished by temporarily deforming the opening 28 and retainer 32 and without removing portions of the housing or otherwise damaging the housing 12-1. The magnet apparatus 34-1 (FIGS. 11, 23 and 24) may be then be installed into the same pocket 26-1 in place of the removed magnet system 100a by way of the same opening 28 (Step 02a). The replacement may also be accomplished by temporarily deforming the opening 28 and retainer 32 and without removing portions of the housing or otherwise damaging the housing 12. Suitable removal and installation tools and techniques are illustrated and described in U.S. Pat. No. 10,124,167.

The exemplary magnet system 100a may be configured to fit into the housing pocket 26-1 and, accordingly, may have a diameter D4 (FIG. 13) of about 9.4 mm to about 17.4 mm and the thickness T4 of about 1.5 mm to about 4.0 mm. In the illustrated embodiment, the diameter D4 of the magnet system 100a is about 12.6 mm and the thickness is about 3.1 mm. As used herein in the context of the magnet system 100a, the word "about" means ± 10%.

Another exemplary magnet system is generally represented by reference numeral 100b in FIGS. 19-22. The exemplary magnet system 100b is substantially similar to magnet system 100 and similar elements are represented by similar reference numerals. Here, however, the magnet system 100b is configured to be placed into a larger pocket than the magnet system 100 and the spacer is located within the case. In particular, the exemplary magnet system 100b includes a positioning magnet 102b, including the above-described magnetic disk 104 and a hermetically sealed case 106b, and a non-magnetic spacer (or "spacer") 108b. The magnetic disk 104 and spacer 108b are both located within the case 106b. The case 106b is disk-shaped and may be formed from the same material as the case 106. The magnetic disk 104 and spacer 108b together define an overall disk-like shape that fits into the case. To that end, the spacer 108b includes a base 110b and an annular side wall 112b that together define a receptacle 114b. The inner diameter of the side wall 112b is substantially equal to the outer diameter of the magnetic disk 104, while the depth of the receptacle 114b is substantially equal to the thickness of the magnetic disk. Suitable materials for the exemplary spacer 108b, which may be formed by machining, metal injection molding or injection molding, include paramagnetic metals, polymers and plastics such as those discussed above in the context of the case 106.

The size and shape of the magnet system 100b may be substantially equal to the cochlear implant magnet pocket that it is intended to be inserted into during, for example, an in-situ retrofit. Turning to FIGS. 22 and 22A, the exemplary magnet system 100b may be the same size, or essentially the same size, as the pocket 26-1. As such, the magnet system 100b may be used in an in-situ retrofit procedure where, for example, it is desired to replace a magnet apparatus such as magnet apparatus 34-1 (FIGS. 11, 23 and 24) with the positioning magnet 102b despite the fact that the positioning magnet 102b is smaller than both the pocket 26-1 and the removed magnet apparatus 34-1 with five rotating magnets 44. Referring to FIG. 22B, the magnet apparatus 34-1 may be removed from the pocket 26-1 in-situ by way of the opening 28 in the housing 12-1 (Step 01). The removal may be accomplished by temporarily deforming the opening 28 and retainer 32 and without removing portions of the housing or otherwise damaging the housing 12-1. The magnet system 100b may then be installed into the same pocket 26-1 in place of the removed magnet apparatus 34-1 by way of the same opening 28 (Step 02). The replacement may also be accomplished by temporarily deforming the opening 28 and retainer 32 and without removing portions of the housing or otherwise damaging the housing 12-1. Suitable removal and installation tools and techniques are illustrated and described in U.S. Pat. No. 10,124,167.

As noted above, the diameter D3 of the pocket 26-1 may range from 9.4 mm to 17.4 mm and the thickness T3 may range from 1.5 mm to 4.0 mm and, in the illustrated embodiment, the diameter D3 is 12.6 mm and the thickness T3 is 3.1 mm. Accordingly, the diameter D5 of the magnet system 100b (FIG. 22) may range from about 9.4 mm to about 17.4 mm and the thickness T5 may range from about 1.5 mm to about 4.0 mm. In the illustrated embodiment, the diameter D5 of the magnet system 100b is about 12.6 mm and the thickness T5 is about 3.1 mm. As used herein in the context of the magnet system 100b, the word "about" means ± 10%.

A wide variety of MRI-compatible magnet apparatus may be used to provide cochlear implants with different levels of magnetic strength. Referring first to FIGS. 23 and 24, and as noted above, the exemplary MRI-compatible magnet apparatus 34-1 includes a case 36-1, with a base 38-1 and a cover 40-1, a magnet frame 42-1, and a plurality of elongate diametrically magnetized magnets 44 within the frame.

The case 36-1 is disk-shaped and defines a central axis A1, which is also the central axis of the frame 42-1. The frame 42-1 is rotatable relative to the case 36-1 about the central axis A1 over 360°. The magnets 44 rotate with the frame 42-1 about the central axis A1. Each magnet 44 is also rotatable relative to the frame 42-1 about its own longitudinal axis A2 (also referred to as "axis A2") over 360°. In the illustrated implementation, the longitudinal axes A2 are parallel to one another and are perpendicular to the central axis A1. In other implementations, the magnets may be oriented such that the longitudinal axes thereof are at least substantially perpendicular to the central axis A1. As used herein, an axis that is "at least substantially perpendicular to the central axis" includes axes that are perpendicular to the central axis as well as axes that are slightly non-perpendicular to the central axis (i.e., axes that are offset from perpendicular by up to 5 degrees).

The exemplary case 36-1 is not limited to any particular configuration, size or shape. In the illustrated implementation, the case 36-1 is a two-part structure that includes the base 38-1 and the cover 40-1 which are secured to one another in such a manner that a hermetic seal is formed between the cover and the base. Suitable techniques for securing the cover 40-1 to the base 38-1 include, for example, seam welding with a laser welder. The case 36-1 may be formed from the material described above with reference to case 106.

The exemplary frame 42-1 includes a disk 43-1 and a receptacle 46-1 that extends completely through the disk. Suitable materials for the frame 42-1, which may be formed by machining, metal injection molding or injection molding, include paramagnetic metals, polymers and plastics such as those discussed above in the context of the case 106.

The magnets 44 in the exemplary magnet apparatus 34-1 are elongate diametrically magnetized magnets that are circular in a cross-section that is perpendicular to the longitudinal axis A1 and, in some instances, may have rounded corners. There are five magnets 44 and, as compared to one another, three of the magnets are relatively long and two are relatively short. Suitable materials for the magnets 44 include, but are not limited to, neodymium-boron-iron and samarium-cobalt. The magnets 44 may be located within tubes 48 formed from low friction material. Suitable materials for the tubes 48 include polymers, such as silicone, PEEK and other plastics, PTFE, and PEEK-PTFE blends, and paramagnet metals. The magnets 44 may be secured to the tubes 48 such that the each tube rotates with the associated magnet about its axis A2, or the magnets may be free to rotate relative to the tubes. The magnet/tube combination is also more mechanically robust than a magnet alone. The magnets 44 may, in place of the tubes 48, be coated with the lubricious materials discussed below.

Friction may be further reduced by coating the inner surfaces of the case 36-1 and/or the surfaces of the frame 42-1 with a lubricious layer (not shown). The lubricious layer may be in the form of a specific finish of the surface that reduces friction, as compared to an unfinished surface, or may be a coating of a lubricious material such as diamond-like carbon (DLC), titanium nitride (TiN), PTFE, polyethylene glycol (PEG), Parylene, fluorinated ethylene propylene (FEP) and electroless nickel sold under the tradenames Nedox^{®} and Nedox PF^{™}. The DLC coating, for example, may be only 0.5 to 5 microns thick. In those instances where the base 38-1 and a cover 40-1 are formed by stamping, the finishing process may occur prior to stamping. Micro-balls, biocompatible oils and lubricating powders may also be added to the interior of the case to reduce friction.

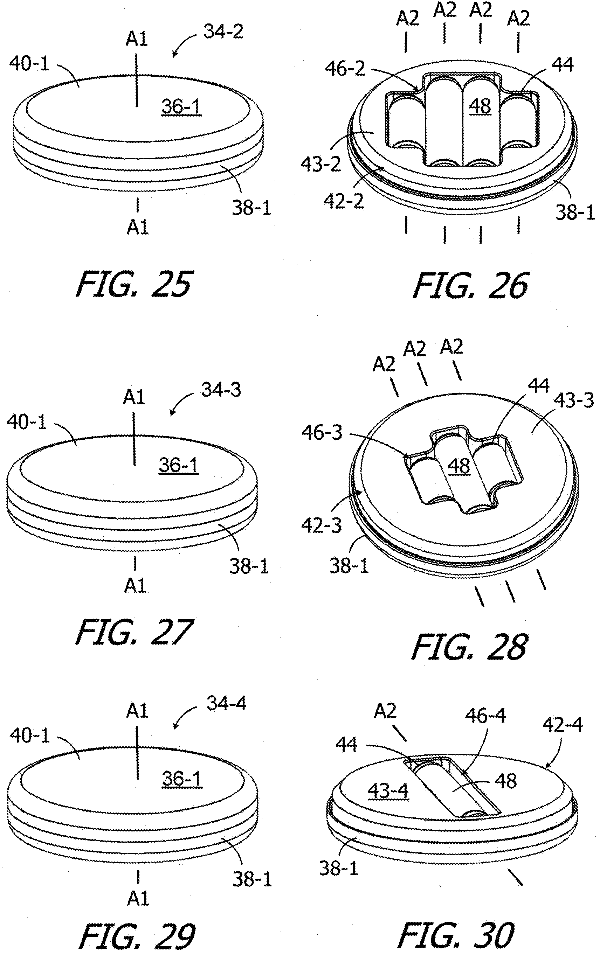

Another exemplary MRI-compatible magnet apparatus, which is generally represented by reference numeral 34-2, is illustrated in FIGS. 25 and 26. Magnet apparatus 34-2 is substantially similar to magnet apparatus 34-1 and similar elements are represented by similar reference numerals. For example, the magnet apparatus 34-2 includes a case 36-1, with a base 38-1 and a cover 40-1, a magnet frame 42-2, with a disk 43-2 and a receptacle 46-2, and a plurality of elongate diametrically magnetized magnets 44 within the frame. The same materials and coatings may also be employed. Here, however, there are only four magnets 44 and magnets occupy less of the interior volume of the case 36-1 than do the five magnets 44 of the magnet apparatus 34-1. The magnetic strength of the magnet apparatus 34-2 is, therefore, less than the magnetic strength of the magnet apparatus 34-1 despite the fact that the magnet apparatus 34-2 is the same size as magnet apparatus 34-1. As such, the MRI-compatible magnet apparatus 34-2 can be used to provide a lower level of magnetic strength, as compared to the magnet apparatus 34-1, with the same sized apparatus. The magnetic strength of an implanted cochlear implant may, therefore, be adjusted in-situ by removing one magnet apparatus and replacing it with a magnet apparatus of a different strength.

Another exemplary MRI-compatible magnet apparatus, which is generally represented by reference numeral 34-3, is illustrated in FIGS. 27 and 28. Magnet apparatus 34-3 is substantially similar to magnet apparatus 34-1 and similar elements are represented by similar reference numerals. For example, the magnet apparatus 34-3 includes a case 36-1, with a base 38-1 and a cover 40-1, a magnet frame 42-3, with a disk 43-3 and a receptacle 46-3, and a plurality of elongate diametrically magnetized magnets 44 within the frame. The same materials and coatings may also be employed. Here, however, there are only three magnets 44 and magnets occupy less of the interior volume of the case 36-1 than do the four magnets 44 of the magnet apparatus 34-2 (FIGS. 25 and 26). The magnetic strength of the magnet apparatus 34-3 is, therefore, less than the magnetic strength of the magnet apparatus 34-1 and the magnet apparatus 34-2 despite the fact that the magnet apparatus 34-3 is the same size as magnet apparatus 34-1 and magnet apparatus 34-2. As such, the MRI-compatible magnet apparatus 34-3 can be used to provide a lower level of magnetic strength, as compared to the magnet apparatus 34-1 or 34-2, with the same sized apparatus. The magnetic strength of an implanted cochlear implant may, therefore, be adjusted in-situ by removing one magnet apparatus and replacing it with a magnet apparatus of a different strength.

Another exemplary MRI-compatible magnet apparatus, which is generally represented by reference numeral 34-4, is illustrated in FIGS. 29 and 30. Magnet apparatus 34-3 is substantially similar to magnet apparatus 34-1 and similar elements are represented by similar reference numerals. For example, the magnet apparatus 34-3 includes a case 36-1, with a base 38-1 and a cover 40-1, a magnet frame 42-4, with a disk 43-4 and a receptacle 46-4. The same materials and coatings may also be employed. Here, however, there is only one elongate diametrically magnetized magnet 44 within the frame and the magnet occupies less of the interior volume of the case 36-1 than do the three magnets 44 of the magnet apparatus 34-3 (FIGS. 27 and 28). The magnetic strength of the magnet apparatus 34-4 is, therefore, less than the magnetic strength of the magnet apparatuses 34-1 to 34-3 despite the fact that the magnet apparatus 34-4 is the same size as magnet apparatuses 34-1 to 34-3. As such, the MRI-compatible magnet apparatus 34-4 can be used to provide a lower level of magnetic strength, as compared to the magnet apparatuses 34-1 to 34-3, with the same sized apparatus. The magnetic strength of an implanted cochlear implant may, therefore, be adjusted in-situ by removing one magnet apparatus and replacing it with a magnet apparatus of a different strength.

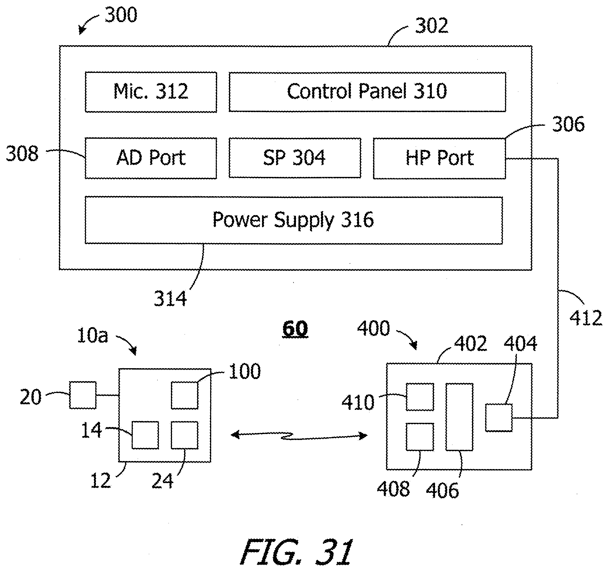

Turning to FIG. 31, the exemplary cochlear implant system 60 may include the cochlear implant 10a (or a cochlear implant with of the magnet apparatuses or systems described herein), a sound processor, such as the illustrated body worn sound processor 300 or a behind-the-ear sound processor, and a headpiece 400.

The exemplary body worn sound processor 300 in the exemplary ICS system 60 includes a housing 302 in which and/or on which various components are supported. Such components may include, but are not limited to, sound processor circuitry 304, a headpiece port 306, an auxiliary device port 308 for an auxiliary device such as a mobile phone or a music player, a control panel 310, one or more microphones 312, and a power supply receptacle 314 for a removable battery or other removable power supply 314 (e.g., rechargeable and disposable batteries or other electrochemical cells). The sound processor circuitry 304 converts electrical signals from the microphone 312 into stimulation data. The exemplary headpiece 400 includes a housing 402 and various components, e.g., a RF connector 404, a microphone 406, an antenna (or other transmitter) 408 and an axially magnetized disk-shaped positioning magnet 410, that are carried by the housing. The headpiece 400 may be connected to the sound processor headpiece port 306 by a cable 412. The external positioning magnet 410 is attracted to the positioning magnet 102 of the cochlear implant 10a (FIG. 10), thereby aligning the antenna 408 with the antenna 24. The stimulation data and, in many instances power, is supplied to the headpiece 400. The headpiece 400 transcutaneously transmits the stimulation data, and in many instances power, to the cochlear implant 10a by way of a wireless link between the antennas. The stimulation processor 14 converts the stimulation data into stimulation signals that stimulate the electrodes 22 of the electrode array 20.

In at least some implementations, the cable 412 will be configured for forward telemetry and power signals at 49 MHz and back telemetry signals at 10.7 MHz. It should be noted that, in other implementations, communication between a sound processor and a headpiece and/or auxiliary device may be accomplished through wireless communication techniques. Additionally, given the presence of the microphone(s) 312 on the sound processor 300, the microphone 406 may be also be omitted in some instances.

The functionality of the sound processor 300 and headpiece 400 may also be combined into a single head wearable sound processor that includes all of the external components (e.g., the battery, microphone, sound processor, antenna coil and magnet). Examples of head wearable sound processors are illustrated and described in U.S. Patent Nos. 8,811,643 and 8,983,102. Headpieces and head wearable sound processors are collectively referred to herein as "head wearable external components."

Although the invention disclosed herein has been described in terms of the preferred embodiments above, numerous modifications and/or additions to the above-described preferred embodiments would be readily apparent to one skilled in the art. The invention includes any combination of the elements from the various species and embodiments disclosed in the specification that are not already described. It is intended that the scope of the present invention extends to all such modifications and/or additions and that the scope of the present invention is limited solely by the claims set forth below.

## Claims

1. A cochlear implant, comprising:
a cochlear lead (16) including a plurality of electrodes (22);
a housing (12) including a magnet pocket (26) defining a magnet pocket diameter (D1, D3), a top wall (30) above the magnet pocket (26), a bottom wall that does not include an opening below the magnet pocket (26), and a magnet aperture (28) that extends through the top wall (30) to the magnet pocket (26);
an antenna (24) within the housing (12) and adjacent to the magnet pocket (26); and
a stimulation processor (14) within the housing (12) operably connected to the antenna (24) and to the cochlear lead (16);
wherein
the cochlear implant further comprises either
a first magnet system (100, 100a), located within the magnet pocket (26) and removable from the pocket (26) by way of the magnet aperture (28), including a non-magnetic spacer (108, 108a) including a receptacle (114, 114a) and defining an outer diameter (D2, D4) that is substantially equal to the magnet pocket diameter (D1, D3), a hermetically sealed case (106, 36) located within the receptacle (114, 114a) and permanently secured to the non-magnetic spacer (108, 108a), and at least one magnet (104, 44) located within the hermetically sealed case (106, 36), or
a second magnet system (100b), located within the magnet pocket (26) and removable from the pocket (26) by way of the magnet aperture (28), including a hermetically sealed case (106b) defining an outer diameter (D5) that is substantially equal to the magnet pocket diameter (D3), a non-magnetic spacer (108b) located within the hermetically sealed case (106b) and including a receptacle (114b), and an axially magnetized magnetic disk (104) that is located within the receptacle (114b).

2. A cochlear implant as claimed in claim 1, wherein
the non-magnetic spacer (108, 108b) includes a base (110, 110b) and an annular side wall (112, 112b) that together define the receptacle (114, 114b).

3. A cochlear implant as claimed in claim 1, wherein
the cochlear implant includes the first magnet system (100a); and
the non-magnetic spacer (108a) includes a top portion (112a) and bottom portion (110a) that together define the receptacle (114a).

4. A cochlear implant as claimed in claim 1, wherein
the cochlear implant includes the first magnet system (100, 100a); and
the at least one magnet comprises an axially magnetized magnetic disk.

5. A cochlear implant as claimed in claim 1,
the cochlear implant includes the first magnet system (100a); and
a frame (42) within the hermetically sealed case (36);
wherein
the hermetically sealed case (36) defines a central axis (A1);
the frame (42) is rotatable relative to the hermetically sealed case (36) about the central axis (A1) of the hermetically sealed case (36); and
the at least one magnet comprises a plurality of elongate diametrically magnetized magnets (44) that are located in the frame (42) and are rotatable relative to the frame (42).

## Patentansprüche

1. Cochleaimplantat mit:
einer Cochleazuleitung (16) mit einer Mehrzahl von Elektroden (22);
einem Gehäuse (12) mit einer Magnettasche (26) mit einem Magnettaschendurchmesser (D1, D3), einer oberen Wand (30) oberhalb der Magnettasche (26), einer Bodenwand, welche keine Öffnung unterhalb der Magnettasche (26) beinhaltet, sowie einer Magnetöffnung (28), welche sich durch die obere Wand (30) zu der Magnettasche (26) hin erstreckt;
einer Antenne (24) innerhalb des Gehäuses (12) und benachbart zu der Magnettasche (26); und
einem Stimulationsprozessor (14) innerhalb des Gehäuses (12), welcher in Wirkverbindung mit der Antenne (24) und der Cochleazuleitung (16) steht;
wobei das Cochleaimplantat versehen ist entweder mit
einem ersten Magnetsystem (100, 100a), welches innerhalb der Magnettasche (26) angeordnet ist und mittels der Magnetöffnung (28) aus der Tasche (26) entfernbar ist, mit einem nicht-magnetischen Abstandshalter (108, 108a) mit einer Aufnahme (114, 114a) und einem Außendurchmesser (D2, D4), der im Wesentlichen gleich dem Magnettaschendurchmesser (D1, D3) ist, einem hermetisch abgedichteten Gehäuse (106, 36), welches innerhalb der Aufnahme (114, 114a) angeordnet ist und permanent mit dem nicht-magnetischen Abstandshalter (108, 108a) fest verbunden ist, sowie mindestens einem Magneten (104, 44), der innerhalb des hermetisch abgedichteten Gehäuses (106, 36) angeordnet ist; oder
einem zweiten Magnetsystem (100b) welches innerhalb der Magnettasche (26) angeordnet ist und mittels der Magnetöffnung (28) aus der Tasche entfernbar ist, mit einem hermetisch abgedichteten Gehäuse (106b) mit einem Außendurchmesser (D5), der im Wesentlichen gleich dem Magnettaschendurchmesser (D3) ist, einem nicht-magnetischen Abstandshalter (108d), der innerhalb des hermetisch abgedichteten Gehäuses (106b) angeordnet ist und eine Aufnahme (114b) aufweist, sowie einer axial magnetisierten magnetischen Scheibe (104), welche innerhalb der Aufnahme (114b) angeordnet ist.

2. Cochleaimplantat gemäß Anspruch 1, wobei der nicht-magnetische Abstandshalter (108, 108b) eine Basis (110, 110b) sowie eine ringförmige Seitenwand (112, 112b) aufweist, welche zusammen die Aufnahme (114, 114b) bilden.

3. Cochleaimplantat gemäß Anspruch 1, wobei das Cochleaimplantat das erste Magnetsystem (100a) beinhaltet und der nicht-magnetische Abstandshalter (108a) einen oberen Abschnitt (112a) und einen Bodenabschnitt (110a) aufweist, welche zusammen die Aufnahme (114a) festlegen.

4. Cochleaimplantat gemäß Anspruch 1, wobei das Cochleaimplantat ein erstes Magnetsystem (100, 100a) aufweist, und der mindestens eine Magnet eine axial magnetisierte magnetische Scheibe aufweist.

5. Cochleaimplantat gemäß Anspruch 1, wobei das Cochleaimplantat das erste Magnetsystem (100a) und einen Rahmen (42) innerhalb des hermetisch abgedichteten Gehäuses (36) aufweist;
wobei
das hermetisch abgedichtete Gehäuse (36) eine zentrale Achse (A1) aufweist;
der Rahmen (42) um die zentrale Achse (A1) des hermetisch abgedichteten Gehäuses (36) relativ zu dem hermetisch abgedichteten Gehäuse (36) drehbar ist; und
der mindestens eine Magnet eine Mehrzahl von langgestreckten diametral magnetisierten Magneten (44) aufweist, die in dem Rahmen (42) angeordnet sind und relativ zu dem Rahmen (42) drehbar sind.

## Revendications

1. Implant cochléaire, comprenant :
un fil cochléaire (16) comportant une pluralité d'électrodes (22) ;
un logement (12) comportant une poche d'aimant (26) définissant un diamètre de poche d'aimant (D1, D3), une paroi supérieure (30) au-dessus de la poche d'aimant (26), une paroi inférieure qui ne comprend pas d'ouverture sous la poche d'aimant (26) et une ouverture d'aimant (28) qui s'étend à travers la paroi supérieure (30) jusqu'à la poche d'aimant (26) ;
une antenne (24) à l'intérieur du logement (12) et adjacente à la poche d'aimant (26) ; et
un processeur de stimulation (14) à l'intérieur du logement (12) connecté fonctionnellement à l'antenne (24) et au fil cochléaire (16) ;
l'implant cochléaire comprenant en outre soit un premier système d'aimant (100, 100a), situé à l'intérieur de la poche d'aimant (26) et pouvant être retiré de la poche (26) via l'ouverture d'aimant (28),
comportant une entretoise non magnétique (108, 108a) comportant un réceptacle (114, 114a) et définissant un diamètre extérieur (D2, D4) sensiblement égal au diamètre de la poche d'aimant (D1, D3), un boîtier hermétiquement fermé (106, 36) situé à l'intérieur du réceptacle (114, 114a) et fixé de manière permanente à l'entretoise non magnétique (108, 108a) et au moins un aimant (104, 44) situé à l'intérieur du boîtier hermétiquement fermé (106, 36), soit
un second système d'aimant (100b), situé à l'intérieur de la poche d'aimant (26) et pouvant être retiré de la poche (26) via l'ouverture d'aimant (28), comportant un boîtier hermétiquement fermé (106b) définissant un diamètre extérieur (D5) sensiblement égal au diamètre de la poche d'aimant (D3), une entretoise non magnétique (108b) située à l'intérieur du boîtier hermétiquement fermé (106b) et comportant un réceptacle (114b), et un disque magnétique axialement aimanté (104) situé à l'intérieur du réceptacle (114b).

2. Implant cochléaire selon la revendication 1,
l'entretoise non magnétique (108, 108b) comprenant une base (110, 110b) et une paroi latérale annulaire (112, 112b) qui définissent ensemble le réceptacle (114, 114b).

3. Implant cochléaire selon la revendication 1,
l'implant cochléaire comprenant le premier système d'aimant (100a) ; et
l'entretoise non magnétique (108a) comprenant une partie supérieure (112a) et une partie inférieure (110a) qui définissent ensemble le réceptacle (114a).

4. Implant cochléaire selon la revendication 1,
l'implant cochléaire comprenant le premier système d'aimant (100, 100a) ; et
l'au moins un aimant comprenant un disque magnétique à aimantation axiale.

5. Implant cochléaire selon la revendication 1,
l'implant cochléaire comprenant le premier système d'aimant (100a) ; et
un cadre (42) à l'intérieur du boîtier hermétiquement fermé (36) ;
dans lequel
le boîtier hermétiquement fermé (36) définit un axe central (A1) ;
le cadre (42) est mobile en rotation par rapport au boîtier hermétiquement fermé (36) autour de l'axe central (A1) du boîtier hermétiquement fermé (36) ; et l'au moins un aimant comprend une pluralité d'aimants allongés (44) magnétisés diamétralement qui sont situés dans le cadre (42) et qui peuvent tourner par rapport au cadre (42).
